Europäisches Patentamt

(19) European Patent Office

Office européen des brevets

(11) Publication number: **0 243 179 B1**

(12) **EUROPEAN PATENT SPECIFICATION**

(45) Date of publication of patent specification: **28.10.92**

(21) Application number: **87303549.7**

(22) Date of filing: **22.04.87**

(51) Int. Cl.⁵: **A61K 37/36**, A61K 37/12,
//(A61K37/36,37:12,31:725)

The file contains technical information submitted
after the application was filed and not included in
this specification

(54) **Wound healing composition.**

(30) Priority: **23.04.86 US 855508**
**23.06.86 US 877266**
**12.08.86 US 895757**

(43) Date of publication of application:
**28.10.87 Bulletin 87/44**

(45) Publication of the grant of the patent:
**28.10.92 Bulletin 92/44**

(84) Designated Contracting States:
**AT BE CH DE ES FR GB GR IT LI LU NL SE**

(56) References cited:
**EP-A- 0 105 014**
**EP-A- 0 154 447**

(73) Proprietor: **CELTRIX PHARMACEUTICALS, INC.**
**2500 Faber Place**
**Palo Alto CA 94303(US)**

Proprietor: **THE BOARD OF REGENTS OF THE**
**UNIVERSITY OF WASHINGTON**
**201 Administration Building, The Graduate**
**School AG-10**
**Seattle, Washington 98195(US)**

(72) Inventor: **Sundsmo, John S.**
**2369 Redberry Court**
**Pleasanton, CA 94566(US)**
Inventor: **Ksander, George A.**
**437 Northumberland Avenue**
**Redwood City, CA 94061(US)**
Inventor: **McPherson, John M.**
**764 Sequoia Drive**
**Sunnyvale, CA 94086(US)**
Inventor: **Ross, Russel**
**4811 Northeast 42nd Street**
**Seattle, WA 98105(US)**
Inventor: **Sprugel, Katherine H.**
**1915 Northeast 115th Street**
**Seattle, WA 98125(US)**

(74) Representative: **Goldin, Douglas Michael et al**
**J.A. KEMP & CO. 14, South Square Gray's Inn**
**London WC1R 5EU(GB)**

**Description**

Technical Field

The invention is in the fields of medicine, surgery, and biochemistry. More particularly, it concerns wound healing compositions comprising reconstituted fibrillar collagen, heparin and chemotactic, growth or differentiation factor(s).

Background

The process of wound healing consists of a chronological sequence of events characterized by the various cellular infiltrates that appear within the wound. Immediately after wounding, the process of coagulation involves both the humoral aspects of coagulation and the cellular response. The principal cellular response concerns the interaction of platelets with coagulation proteins, thrombin and collagen. Once the coagulation process is completed, various types of leukocytes appear in the wound in an orderly and reproducible sequence. Fibroblasts, endothelial cells, and capillaries appear in the wound slightly later than leukocytes. The fibroblasts are responsible for the formation of the connective tissue components, specifically collagen and the proteoglycans, and, at a much later stage, for the formation of elastic fibers.

Wound healing is thus a complex process at the cellular level involving primarily fibroblasts and epithelial cells with additional effects being exerted by platelets, macrophages, neutrophils, endothelial cells, myofibroblasts, and perhaps other unidentified cell types. At a biochemical level the processes involved in wound healing are poorly understood, although it is clear that growth factors such as transforming growth factors (TGF-$\alpha$, TGF-$\beta$), platelet-derived growth factor (PDGF), fibroblast growth factor (FGF), epidermal growth factor (EGF), connective tissue activating factors (CTAPs such as CTAP-III) exert effects on these cells in vitro. Topically applied preparations of PDGF or FGF to full thickness wounds in a hamster model did not, however, exhibit any significant stimulation of wound healing ((1986) J. Dermatol. Srug. Oncol. 11:6, pp. 617-625).

Additional studies of growth factors in wound healing models include (1983) Science 219:1329-1331; (1986) Ann. Surg. 203:142-147: (1985) J. Clin. Invest. 76:2323-2329; (1982) Japan. J. Pharmacol. 32:198-201; (1982) J. Surg. Res. 33:164-169; (1985) Proc Natl. Acad. Sci. USA 82:7340-7344; (1985) J. Cell Biol. 100:1219-1227; (1980) Proc. Natl. Acad. Sci. USA 77:4379-4381; (1982) J. Neurosci. Res. 8:413-417; (1985) Exp. Mol. Pathol. 43:274-281; (1986) J. Surg. Res. 40:315-319.

It is known that platelets contain growth factors but it has not been determined whether these factors are normally released at wound sites. Collagen is known to induce platelet aggregation and degranulation reactions in vitro, but again it is not clear whether these reactions also occur in vivo at local wound sites. In sum, a comprehensive biochemical analysis of wound healing has not been accomplished, although it is presumed that the in vitro biological activities of platelets are operative in vivo.

Several prior patent publications have suggested using combinations of collagen and glycosaminoglycans to promote wound healing. Yannas et al have issued a series of U.S. patents describing laminated composites that may be used as synthetic skin. The first of the series, U.S. 4,060,081 describes a composite in which the bottom (skin side) layer is collagen cross-linked with a glycosaminoglycan. The glycosaminoglycan is added to solubilized collagen to form a precipitate, the precipitate is homogenized and then cross-linked with glutaraldehyde. U.S.-A-4,350,629 describes a variation of this process in which the glutaraldehyde is added before the glycosaminoglycan is added to the collagen. U.S.-A-4,418,691 describes yet another variation in which the collagen-glycosaminoglycan lattice is impregnated with viable epithelial, mesenchymal, or fibroblast cells. The patent states the cells may be treated beforehand with substances, such as EGF and PDGF, that increase cell reproduction.

European Patent Application 85301127 (published 11 September 1985 under EP-A-154447) describes a wound healing composition that consists of a suspension of collagen and a glycosaminoglycan such as heparin or heparan sulfate that induces chemotaxis.

The roles of FGF and PDGF in wound healing are described in Growth and Maturation Factors (1986), John Wiley & Sons, Inc., pp. 1-36, and Cell (1986) 46: 155-169, respectively.

The present invention provides a novel wound healing composition whose biological activity is superior to the suspensions described in the mentioned European Patent Application.

Disclosure of the Invention

The present invention is a soft tissue wound healing composition comprising a mixture of:

(a) fibrillar collagen;

(b) 0.1% to 10% by weight based on collagen of heparin a heparin-like glycosaminoglycan, or mixtures thereof; and

(c) an effective amount of at least one chemotactic, growth or differentiation factor.

Preferred factors are PDGF, FGF, or mixtures thereof.

Wound dressings comprising the above composition carried on a solid carrier is another aspect of the invention.

Brief Description of the Drawings

In the drawings:

Figure 1 is a set of bar graphs of results of the histological examinations reported infra showing the fibroplasia evident in histological sections of wound sites treated with various test compositions.

Figure 2 is a set of bar graphs of results of the histological examination reported infra showing the vascularization evident in histological sections of wound sites treated with various test compositions.

Figure 3 is a set of bar graphs of results of the histological examination reported infra showing the re-epithelialization evident in histological sections of wound sites treated with various test compositions.

Figure 4 is a set of bar graphs of results of the histological examination reported infra showing total epithelial length (in ocular micrometer units) evident in histological sections of wound sites treated with various test compositions.

Modes of Carrying Out the Invention

The composition of the invention is useful for treating soft tissue wounds such as cutaneous, dermal, mucosal or epithelial wounds in vertebrates. It is especially useful for treating cutaneous wounds in mammals including man, domestic and farm animals, sports animals, and pets. It may be used to treat any type of full or partial thickness cutaneous wounds including traumatic wounds, surgical wounds, thermal or chemical wounds (burns) radiation wounds and chronic ulcers such as decubiti, and cutaneous ulcers caused by vascular, hematologic and metabolic diseases, infections, or neoplasms.

The collagen that is used in the invention is fibrillar and is capable of binding heparin or heparin-like glycosaminoglycans. Type I or Type III collagen, or mixtures thereof, are preferred because of their heparin binding capacity. The collagen may be of genetically dissimilar origin (e.g., allogeneic or xenogeneic) than the individual to which it is applied. If the collagen is xenogeneic it is preferred that it be purified (e.g., by enzymatic or other treatment to remove antigenic determinants) to lessen or reduce the likelihood of immunogenicity. The collagen fibers are reconstituted and may be cross-linked or noncross-linked. Reconstituted bovine dermal fibrillar collagen suitable for use in the composition is available commercially under the trademark ZYDERM® from Collagen Corporation, Palo Alto, California. When in the form of an aqueous suspension, the fibrillar collagen concentration is in the range of about 1 to 70 mg/ml. A collagen concentration range of 15 to 35 mg/ml is preferred for handling purposes Depending upon the concentration of collagen in the suspension the consistency of the suspension will range from a translucent gel to a runny syrup.

Heparin or a heparin-like glycosaminoglycan is mixed with the fibrillar collagen in amounts ranging between about 0.1% to about 10% by weight, based on collagen, preferably about 0.3% to 3%, and most Preferably about 1%. Heparin is a staple product of commerce. Fragments and derivatives of heparin are known which possess chemical similarity to heparin. As used herein the term "heparin-like glycosaminoglycan" is intended to include such fragments and derivatives, provided they are functionally equivalent to heparin in the composition (i.e., combine with collagen and the factor(s) to provide efficacious wound healing).

The chemotactic, growth or differentiation factors used in the invention may be native or synthetic (recombinant) and may be of human or other mammalian type. Human FGF (either acidic form or basic form) and PDGF are preferred. Other such factors include EGF, TGF-$\alpha$, TGF-$\beta$, and CTAP-III. Methods for isolating FGF from native sources (pituitary, brain) are described in Bohlen et al (1984) Proc. Natl. Acad. Sci. USA 81:5364, and methods for isolating PDGF from platelets are described by Rainer et al (1982) J. Biol. Chem. 257:5154. Kelly et al (1985) EMBO J. 4:3399 discloses procedures for making recombinant forms of PDGF. Synthetic analogs of the factors may be used provided they retain the biological activity of the native molecule. Such analogs are intended to be within the scope of the term "chemotactic, growth or differentiation factor" or the specific terms used to denote particular factors, e.g., "FGF" or "PDGF". Such analogs may be made by conventional genetic engineering such as via expression of synthetic genes or by expression of genes altered by site-specific mutagenesis. In some cases, such as with PDGF, the factor may be incorporated into the composition in its native form (i.e., as platelets), or as crude or partially purified releasates or extracts. Alternatively, the factors may be incorporated in a substantially pure form free of significant amounts of other contaminating materials. The amount of factor included in the composition will depend upon the particular factor involved and its specific activity. In most instances the factor(s) will be present in amounts in the range of 0.01% to 1% based on collagen.

Platelets for use in the invention are isolated from vertebrate blood, preferably mammalian blood, under conditions which prevent degranulation. Such conditions are well known and typically involve mixing the blood with an anticoagulant solution and centrifuging the platelets from the mixture. The mammalian species of the platelets are diluted in buffer and then added to the fibrillar collagen-heparin mixture. The final volume dilution of platelets, expressed in terms of volume of packed platelets, is in the range of 1:10 to 1:10,000, preferably 1:50 to 1:100. Expressed in terms of volume % based on collagen, the packed platelets are added to the mixture at levels in the range of 0.01% to 10% preferably 2% to 10%.

The platelet releasate that may be used in the composition comprises granule constituents that are released after aggregation and that possess angiogenic activity, chemotactic activity, mitogenic activity, connective tissue deposition activity, or epidermal cell proliferation activity. It may be prepared by sonicating platelets or treating a platelet suspension with agents that cause the platelets to aggregate and release granule constituents. Examples of such agents are thrombin, collagen, ADP and immune complexes. After such treatment solids (e.g., cellular debris, aggregated platelets) are separated such as by centrifugation. The releasate may be purified by affinity chromatography using a heparin-Sepharose column. The amount of releasate used in the mixture is that which is equivalent to 0.01% to 10% by volume, preferably 2% to 10%, based on collagen of packed platelets. In other words, one uses that amount of releasate that is derived from such an amount of packed platelets.

In addition to the fibrillar collagen, heparin, and factor(s), the aqueous formulations of the composition will typically contain buffers and salts that maintain the aqueous mixture at approximately physiological pH and ionic strength (i.e., pH 6.8 to 7.4; ionic strength (r/2) 0.1 to 0.2). Minor amounts of other additives such as local anesthetics (e.g., lidocaine) bacteriostats (e.g., sulfadiazine and silver or sodium salts thereof), antibiotics (e.g., ampicillin), gelling agents (e.g., gelatin), or serum proteins may be included in the wound healing composition.

The mixture of fibrillar collagen, heparin, and factor(s) is applied topically to the wound site. The composition may be applied per se or in the form of an occlusive dressing. When applied per se the composition is applied as an ointment, gel, lotion, spray, powder or paste, depending upon its consistency. If the wound is depressed, the composition is packed into the wound. The composition is preferably applied in conjunction with means for keeping the composition at the wound site and, in the case of open wounds, for maintaining the composition hydrated. For cutaneous wounds such means are exemplified by dressings, such as Opsite wound dressing, having a suitable water vapor transmission rate, or perhaps skin grafts or flaps that are placed over the composition.

When applied in the form of an occlusive dressing the composition is impregnated, coated, adsorbed or otherwise applied to a synthetic or natural solid support, such as a fibrous or nonfibrous backing or sponge. and the composite is applied to the wound site.

The following examples further illustrate the invention and its relationship to the prior art.

A. Preparation of Collagen-Heparin-Platelet Composites

Test wound healing compositions were prepared in a stepwise manner. First fibrillar collagen/heparin (FC-H) was prepared by mixing 10 ml of commercially available bovine dermal fibrillar collagen (FC; 35 mg/ml) with 1 ml of 3.3 mg/ml heparin (H). Second, platelets were isolated and purified from 30 ml of blood obtained from Hartley strain guinea pigs by differential centrifugation using standard methods. The final packed undegranulated platelet cell pellet was resuspended in 3 ml of Tyrodes buffered saline (TBS). Third, composites were prepared containing three different dilutions of platelets by: a) mixing 1 ml of platelets with 2 ml of FC-H giving a final platelet dilution of 1/12 (FC-H/PL-12); b) diluting platelets 1/10 with TBS and then mixing 1 ml of platelets with 2 ml FC-H (FC-H/PL-120); or, c) diluting platelets 1/1000 with TBS and then mixing 1 ml of platelets with 2 ml of FC-H (FC-H/PL-1200). Control materials were also prepared containing 1 ml of platelets mixed with 2 ml of FC (rather than FC-H).

B. Preparation of Collagen-Heparin-Platelet Releasate Composites

Platelets were isolated by differential centrifugation using standard methods. The final washed platelet cell pellet was resuspended in 25 ml of 20 mM Tris buffer, PH 7.5, containing 0.14 M NaCl, 15 mM KCl, 5 mM glucose, and 2 mM $CaCl_2$. Thrombin (7 units/ml platelets) was added to induce aggregation and release. After 10 min at room temperature the aggregated platelets were removed by centrifugation at 22610 × g for 20 min at 4°C. The concentration of the platelet proteins in the releasate was determined to be 642 µg/ml by Lowry analysis. Platelet releasate was stored frozen at -70°C until use. This releasate was tested in vitro and found to stimulate human skin fibroplast proliferation.

A portion of the platelet releasate was purified by heparin-Sepharose column chromatography as follows: five ml of platelet releasate was applied to a column (0.7 X 9 cm) of heparin-Sepharose CL-6B which was previously equilibrated in 20 mM Tris buffer, pH 7.6, containing 0.15M NaCl and 1 mM $CaCl_2$. After applying the sample, the column was washed with this same buffer until all the absorbance at 278 nm had reached a background level. Protein bound on the column was eluted with 20 mM Tris, pH 7.6, containing 2.0 M NaCl and 1 mM $CaCl_2$. The resultant flow-through fraction (non-bound fraction) and 2 M NaCl eluate (bound fraction) were collected, pooled, dialyzed to 0.1 M ammonium bicarbonate, pH 8, and lyophilized. Lyophilized samples were resuspended in 20 mM Tris, pH 7.6, containing 1 mM $CaCl_2$ and the insoluble residue was removed by centrifugation. Protein concentration was determined by Lowry analysis.

Releasate or chromatographed releasate is mixed with fibrillar collagen and heparin in proportions equivalent to the packed volumes of platelets used.

### C. Wound Model

Wounds were created in the dermis of Hartley guinea pigs using a 6 mm biopsy punch. These wounds were filled with FC, FC-H, FC-H/PL-12, FC-H/PL-120, or FC-H/PL-1200 composites and the sites were then covered with an occlusive dermal dressing. Wound sites were surgically removed after 5 or 11 days, fixed, embedded, sectioned, and stained for histological examination using hematoxylin-eosin (H&E) or Gomori trichrome stain to visualize collagen.

### D. Histological Evaluation

The following criteria were used to provide numerical evaluation of histological parameters.

The relative number of cells in the wound or subcutaneous implant sites (e.g., fibroblasts, macrophages, eosinophils, lymphocytes, plasma cells, polymorphonuclear neutrophils (PMN), macrophage giant cells, or adipocytes (fat cells) was graded on a scale of 0 to 3+, where 0 corresponded to no cells visible; 1+ was a few scattered cells; 2+ was many scattered cells; 3+ was concentrated masses containing large numbers of cells.

Vascularity of the wound and implant sites was graded on a scale of 0 to 3+, where 0 was no vessels visible; 1+ was several small vessels; 2+ was many small and a few large vessels; and 3+ was many large vessels.

The amount of new host collagen was graded on a scale of 0 to 3+, where new collagen (defined as lightly staining microfibrillar in architecture) filling up to 1/3 of the wound area was graded 1+; new collagen filling I/3 to 2/3 of the wound area was graded 2+; and new collagen filling more than 2/3 of the wound area was graded 3+.

Epithelial maturation was also graded on a scale of 0 to 3+, where thin epithelium composed of flat cells was rated 1+; thicker epithelium with less flattened basal cells and a slight development of the stratum granulosum was graded 2+; and thick epithelium with rounded basal cells, a well developed granulosum, and extensive cornification was graded 3+. An ocular micrometer was used at an objective magnification of 4× to measure the length of the epithelium and the width of wounds. These data are reported in ocular micrometer units where 0.23 mm = 1 ocular unit.

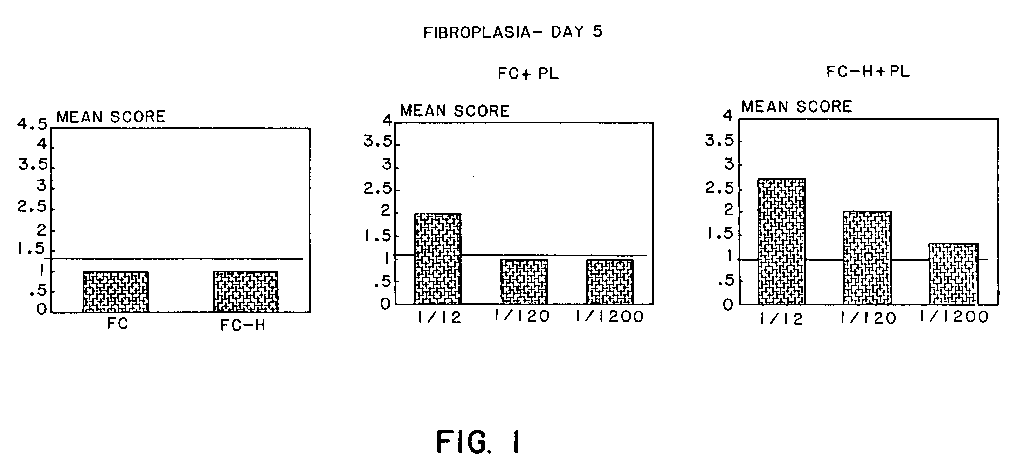

Table 1 summarizes the cellular response observed in the wound sites. The data presented in Table 1 are presented graphically in Figures 1 through 4.

Table 1

Histological and Morphometric Analysis of
Cellularity of Dermal Wounds: Mean Score

| Characteristic | Days | FC-H/PL | | | | FC/PL | | | |
|---|---|---|---|---|---|---|---|---|---|
| | | 1/12 | 1/120 | 1/1200 | None | 1/12 | 1/120 | 1/1200 | None |
| Fibrosis | 5 | 2.7 | 2 | 1.3 | 1 | 2 | 1 | 1 | 1 |
| Vascularity | 5 | 2.3 | 2.3 | 1.7 | 2.7 | 2.3 | 2 | 2 | 1 |
| Epidermal Length (X +/- SE) | 5 | 8.0 / 1.0 | 7.8 / 0.6 | 9.5 / 1.0 | 4.5 / 1.6 | 4.7 / 1.3 | 8.5 / 0.5 | 6.4 / - | 2.7 / 1 |
| Mid-dermal Wound Width (X +/- SE) | 5 | 9.5 / 1.2 | 9.0 / 0.8 | 9.2 / 0.5 | 7.9 / 0.8 | 9.3 / 0.8 | 9.8 / 0.3 | 9.8 / 1.8 | 6.0 / 0 |
| Percentage Epidermis* (X +/- SE) | 5 | 45 / 7 | 43 / 4 | 62 / 8 | 34 / 20 | 22 / 4 | 43 / 4 | 45 / - | 35 / 18 |
| Fibrosis | 11 | 3 | 2.7 | 3 | 2.7 | 2.7 | 2.5 | 2 | 2 |
| Vascularity | 11 | 2.3 | 2.3 | 1.7 | 1.3 | 2 | 2 | 2 | 2 |
| Percentage Epidermis* | 11 | 100 | 100 | 100 | 100 | 100 | 100 | 100 | 100 |
| Epithelial Quality (X +/- SE) | 11 | 2.7 / 0.3 | 2.3 / 0.7 | 2 / 0.6 | 1.7 / 0.3 | 2.7 / 0 | 3 / 0.3 | 1.7 / 0.3 | 1.7 |
| Mid-dermis Wound Width (X +/- SE) | 11 | 10.5 / 0.5 | 7 / 0.3 | 6.7 / 0.2 | 7.8 / 0.6 | 9.5 / 1.3 | 9.8 / 1.3 | 11.3 / 1 | 8.8 / 1.3 |

*Percentage Epidermis = epidermal length/wound width at the surface X 100%.

E. Discussion of Results Shown in Table 1 and Figures 1-4

1. Dermal Wound Sites - Day 5

a. Fibrillar Collagen with Heparin and Platelets (FC-H/PL): Day 5

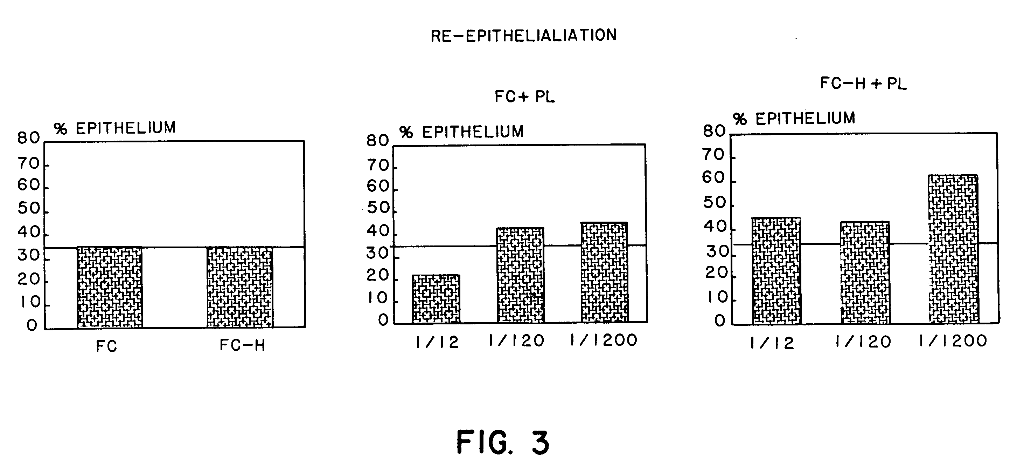

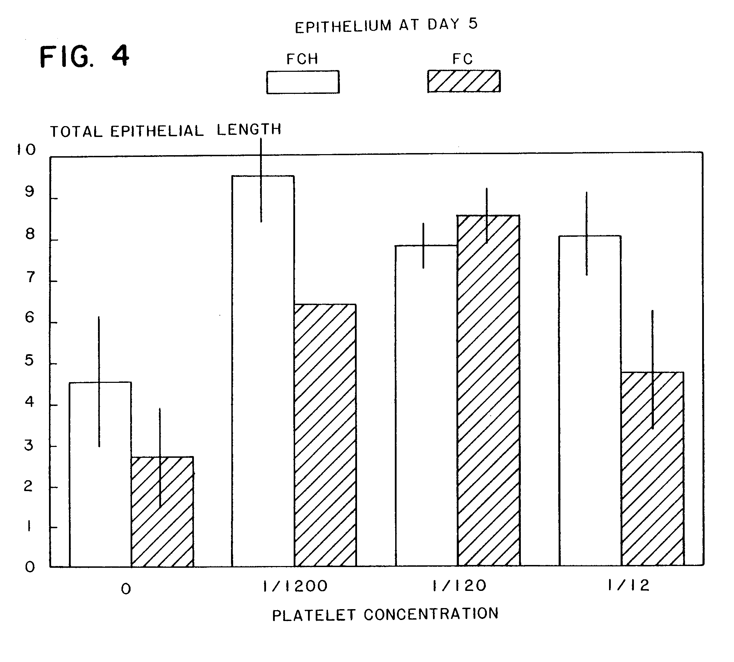

Granulation tissue deposition, fibrosis, new host collagen, and neo-vascularization of wounds was increased at higher doses of platelets (Table 1, Figures 1-2). Morphometric measurements presented in Table 1 and Figure 3 and 4 show the percentage of the wound covered by new epithelium and the total length of newly formed epithelium on day 5. Wounds treated with FC-H/PL had greater amounts of new epithelium than wounds without platelets. This difference was statistically significant (P = .02). The measured difference of the platelet- treated wound sites was also greater than that of the non-platelet treated wounds at the wound surface and at the level of the mid-dermis. It is important that while relatively small percentage differences in re-epithelialization were observed in the FC-H/PL treated wound sites (Figure 3) the actual length of new epithelium was 1.7-2.1 times greater than control FC-H treated sites and 2.9-3.5 times greater than FC treated wound sites. The reason for this apparent disparity in measurements almost certainly lies within the finding of greater mid-dermis measurements in platelet/FC-H treated wounds (Table 1), a finding which suggests less wound contracture in these treated sites and hence a much longer expanse of wound surface which must be covered by new epithelium. Treatment of wound sites with FC-H resulted in granulation tissue deposition which appeared more extensive than that in wound sites treated with FC alone, however, this was not confirmed in morphometric analyses (Table 1, Figure 1). At higher doses of platelets (i.e., 1/12), composite material visible in the tissue sections was broken up into smaller, less dense islands of material through infiltration of fibroblasts.

Inflammation of sites with PMN and lymphocytes was minimal with all composites and the extent of inflammation did not appear related to the dosage of platelets in the wound sites. Giant cells were occasionally observed, however, these were not associated with the implant material, nor were giant cells more prevalent in implant materials formulated with platelets. No eosinophils, plasma cells, or fat cells were observed.

b. Fibrillar Collagen with Platelets (FC/PL): Day 5

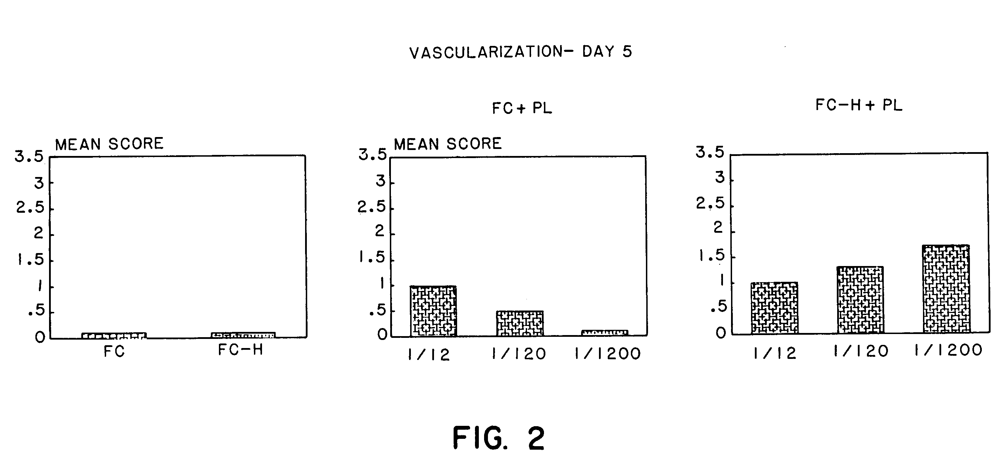

The results also presented in Table 1 summarize the histological findings at wound sites filled with FC with and without platelets. Wounds with higher concentrations of platelets had greater fibroplasia (Figure 1) and more small blood vessels (Figure 2) than controls receiving FC or FC-H alone. Epithelialization was incomplete but the length of new epithelial surface was greater in the platelet containing wounds than in control FC wounds (Table 1, Figures 3-4); FC/PL containing wounds showed greater re-epithelialization than FC-H wounds lacking platelets, but less than FC-H/PL. The FC composite was visible as large, dense and homogeneous masses of collagen with a fine fibrillar substructure. A few clefts or irregular spaces were visible within the composite. The inclusion of platelets in the composite resulted in a separation of the composite into smaller islands of material separated by infiltrating fibroblasts and new collagen. Small numbers of lymphocytes, PMN, or giant cells were seen within the composite. There were no eosinophils, plasma cells, or fat cells visible.

2. Dermal Wound Sites - Day 11

a. Fibrillar Collagen with Heparin and Platelets: Day 11

Histological differences among wounds with different doses of platelets were less pronounced at day 11 than at day 5 (above). All wounds were completely covered by new epithelium and the quality of the epithelium appeared to be related to the number of platelets in the composite. with higher epithelial quality being associated with larger numbers of platelets (Table 1). In these cases the epidermis was thicker, with more rounded basal cells and there was increased differentiation with a more pronounced kerato-hyalin layer and increased cornification. In all wounds less composite was visible than at day 5, and the composite was well infiltrated by fibroblasts. Equivalent amounts of granulation tissue and new collagen were seen in the various groups (Table 1). Wounds treated with higher concentrations of platelets appeared more vascular, however, this was not confirmed in numerical analyses (Table 1). A minimal inflammatory profile was observed: namely, only a few lymphocytes, macrophages, giant cells, and pyknotic neutrophils were observed in these tissue sections.

b. Fibrillar Collagen with Platelets: Day 11

All wounds appeared to be completely re-epithelialized. The quality of the epithelium was notably better in wound sites treated with FC-platelet composites (Table 1). In these cases the epidermis was thicker, with more rounded basal cells, and there was notably greater differentiation of the epidermis with a more pronounced kerato-hyalin layer and increased cornification (Table 1). Fibroplasia and neo-vascularization did not appear to be more pronounced in wound sites treated with FC-platelets than in control wound sites. FC composites were more dispersed at the higher platelet dosages, apparently due to division of the composite by infiltrating fibroblasts. New host collagen synthesis was visible in sites. Small numbers of lymphocytes, giant cells, and pyknotic neutrophils were observed. No plasma cells were noted.

In addition to the histological evaluations, serological evaluations of the animals were made using blood taken at sacrifice and an ELISA for antibodies to the collagen or collagen-platelet composites. No animal developed antibodies to the collagen or the composites. Samples of the composites were also placed in subcutaneous tissue sites in the same animals immediately adjacent the wound sites. The implants were removed after 5 or 11 days. No significant fibroplasia, new collagen formation, inflammation or fibrous

7

encapsulation of the implants were observed thus ruling out the possibility of uncontrolled growth.

In over two years of testing various biomaterials in the guinea pig model described above, the platelet-containing composites were the only materials that have exhibited a significant enhancement in rate of wound healing. The data also show that the composites enhance the extent of wound healing (platelet/FC-H composites inhibit wound contracture). If contracture is related to the formation of keloids or hypertrophic scars, then the composites may provide a mechanism for healing without scarring.

### F. Testing of Collagen-Heparin-Peptide Growth Factor Composites

An aqueous suspension of pure fibrillar collagen (25 mg/ml) (ZYDERM collagen) with or without heparin (250 $\mu$g/ml) was mixed with various peptide growth factors under sterile conditions to give final concentrations of 25 mg/ml collagen, 250 $\mu$g/ml heparin (if included), and 2-8 $\mu$g/ml growth factors. Sterile 1 mm diameter expanded polytetrafluoroethylene tubes (90 $\mu$m pore size) were filled with the collagen/growth factor mixtures and inserted subcutaneously under the abdominal skin of anesthetized adult male Sprague-Dawley rats. After 10 days, the tubes were removed and divided into segments to assay for DNA content and histological evaluation. These measurements provide an indication of the number of cells and blood vessels that have entered and/or proliferated at the site for the purpose of wound repair. DNA contents were measured in duplicate 5 mm segments of tube. Histology was done on paraffin-embedded H/E sections. Cell counts were made at 31 $\times$ grid in separate segments of the same tube. The results of the DNA measurements and histology evaluation are reported in the Table 2 below.

Table 2

| Factor | Matrix | DNA Content (Avg.) (µg/5 mm segment) | Histology |
|---|---|---|---|
| None | collagen | 8.8(25)[1] | ±[2] |
| None | collagen + heparin | 8.0(25) | ± |
| PDGF, 8µg/ml | collagen | 14.5(15) | ± to + |
| PDGF, 8µg/ml | collagen + heparin | 27.2(15) | ++ to +++ |
| TGF-ß 4µg/ml | collagen | 15.6(12) | + to +++ |
| Factor | Matrix | DNA Content (Avg.) (µg/5 mm segment) | Histology |
| TGF-ß 4µg/ml | collagen + heparin | 15.2(15) | ++ to +++ |
| basic FGF 2µg/ml | collagen | 18.3(11) | ± to +++ |
| basic FGF 2µg/ml | collagen + heparin | 22.7(12) | + to ++++ |
| PDGF, 8µg/ml + TGF-ß, 4µg/ml | collagen | 14.0(6) | + to +++ |
| PDGF, 8µg/ml + TGF-ß, 4µg/ml | collagen + heparin | 25.7(6) | +++ |
| PDGF, 8µg/ml + basic FGF 2µg/ml | collagen | 21.4(6) | + to ++++ |
| PDGF, 8µg/ml + basic FGF 2µg/ml | collagen + heparin | 45.2(6) | +++ to ++++ |

```
basic FGF       collagen          21.4(6)                    ++ to +++
   2µg/ml +
TGF-ß
   4µg/ml


basic FGF       collagen +        24.9(6)                       +++
   2µg/ml +       heparin
TGF-ß
   4µg/ml
```

¹( ) = number of replicates.

²

± Few if any cells in collagen or PTFE

+ Some cells in collagen, no capillaries, more cells in PTFE

++ More cells, some capillaries in collagen

+++ Extensive capillaries, lots of cells

++++ Extensive capillaries, absolutely packed with cells

The collagen/heparin compositions containing PDGF alone, FGF alone, or a combination of PDGF and FGF exhibited substantially higher average cell counts than the other compositions tested.

At the concentrations tested, TGF-$\beta$ did not appear to be affected by the presence or absence of heparin and the compositions of TGF-$\beta$, collagen, and heparin did not exhibit the mitogenic or histological activity exhibited by corresponding compositions containing PDGF and/or FGF. In these tests, the combination of PDGF, FGF, collagen and heparin clearly showed the most activity.

**Claims**

**Claims for the following Contracting States : AT, BE, CH, DE, FR, GB, IT, LI, LU, NL, SE**

1. A soft tissue wound healing composition comprising a mixtue of:
   (a) fibrillar collagen;
   (b) 0.1% to 10% by weight based on collagen of heparin, a heparin-like glycosaminoglycan, or mixtures thereof; and
   (c) an effective amount of at least one chemotactic, growth or differentiation factor.

2. A composition according to claim 1 wherein the factor is PDGF, FGF or mixtures thereof.

3. A composition according to claim 1 or 2 wherein the factor is in the form of undegranulated platelets or an equivalent amount of platelet releasate.

4. A composition according to claim 3 wherein the PDGF or FGF is human PDGF or human FGF and the undegranulated platelets or platelet releasate is human platelets or human platelet releasate.

5. A composition according to any one of the preceding claims wherein the fibrillar collagen is Type I collagen, Type III collagen or mixtures thereof.

6. A composition according to any one of the preceding claims wherein the fibrillar collagen is reconstituted fibrillar collagen.

7. A wound dressing comprising a solid support on which is carried a composition according to any one of the preceding claims.

**Claims for the following Contracting States : ES, GR**

1. A method of preparing a soft tissue wound healing composition which comprises combining
   (a) fibrillar collagen,

(b) heparin, a heparin-like glycosaminoglycan or a mixture thereof and

(c) an effective amount of at least one chemotactic, growth or differentiation factor, such that the weight of heparin, heparin-like glycosaminoglycan or a mixture thereof is present at from 0.1% to 10% by weight based on the collagen.

2. A method according to claim 1 wherein the factor is PDGF, FGF or mixtures thereof.

3. A method according to claim 1 or 2 wherein the factor is in the form of undegranulated platelets or an equivalent amount of platelet releasate.

4. A method according to claim 3 wherein the PDGF or FGF is human PDGF or human FGF and the undegranulated platelets or platelet releasate is human platelets or human platelet releasate.

5. A method according to any one of the preceding claims wherein the fibrillar collage in Type I collagen, Type III collagen or mixtures thereof.

6. A method according to any one of the preceding claims wherein the fibrillar collagen is reconstituted fibrillar collagen.

7. A method of preparing a wound dressing comprising applying to a solid support a wound healing composition prepared by the method of any one of the preceding claims.

**Patentansprüche**

**Patentansprüche für folgende Vertragsstaaten : AT, BE, CH, DE, FR, GB, IT, LI, LU, NL, SE**

1. Zusammensetzung zur Wundheilung von weichem Gewebe, umfassend:

(a) fibrilläres Kollagen,

(b) bezogen auf Kollagen, 0,1 bis 10 Gew.-% Heparin, einem heparin-ähnlichen Glycosaminglycan oder einem Gemisch davon und

(c) eine wirksame Menge mindestens eines chemotaktischen Faktors, eines Wachstums- oder eines Differenzierungsfaktors.

2. Zusammensetzung nach Anspruch 1, worin der Faktor PDGF, FGF oder ein Gemisch davon ist.

3. Zusammensetzung nach Anspruch 1 oder 2, worin der Faktor in Form nichtdegranulierter Plättchen oder in einer äquivalenten Menge aus den Plättchen freigesetzten Materials vorliegt.

4. Zusammensetzung nach Anspruch 3, worin der PDGF oder FGF ein Human-PDGF oder Human-FGF ist und die nichtdegranulierten Plättchen oder das aus den Plättchen freigesetzte Material Humanplättchen oder ein aus den Humanplättchen freigesetztes Material sind.

5. Zusammensetzung nach einem der vorhergehenden Ansprüche, worin das fibrilläre Kollagen Kollagen des Typs I, Kollagen des Typs III oder ein Gemisch davon ist.

6. Zusammensetzung nach einem der vorhergehenden Ansprüche, worin das fibrilläre Kollagen rekonstituiertes fibrilläres Kollagen ist.

7. Wundverband, umfassend einen festen Träger, auf den eine Zusammensetzung nach einem der vorhergehenden Ansprüche aufgetragen ist.

**Patentansprüche für folgende Vertragsstaaten : ES, GR**

1. Verfahren zur Herstellung einer Zusammensetzung zur Wundheilung von weichem Gewebe, welches umfaßt: Kombinieren von

(a) fibrillärem Kollagen,

(b) Heparin, einem heparin-ähnlichen Glycosaminglycan oder eines Gemischs davon und

(c) eine wirksame Menge mindestens eines chemotaktischen Faktors, eines Wachstums- oder eines Differenzierungsfaktors,

derart, daß das Gewicht des Heparins, des heparinähnlichen Glycosaminglycans oder eines Gemischs davon, von 0,1 Gew.-% bis 10 Gew.-%, bezogen auf das Kollagen, beträgt.

2. Verfahren nach Anspruch 1, worin der Faktor PDGF, FGF oder ein Gemisch davon ist.

3. Verfahren nach Anspruch 1 oder 2, worin der Faktor in Form nichtdegranulierter Plättchen oder in einer äquivalenten Menge aus den Plättchen freigesetzten Materials vorliegt.

4. Verfahren nach Anspruch 3, worin der PDGF oder FGF ein Human-PDGF oder Human-FGF ist und die nichtdegranulierten Plättchen oder das aus den Plättchen freigesetzte Material Humanplättchen oder aus Humanplättchen freigesetztes Material sind.

5. Verfahren nach einem der vorhergehenden Ansprüche, worin das fibrilläre Kollagen Kollagen des Typs I, Kollagen des Typs III oder ein Gemisch davon ist.

6. Verfahren nach einem der vorhergehenden Ansprüche, worin das fibrilläre Kollagen rekonstituiertes fibrilläres Kollagen ist.

7. Verfahren zum Herstellen eines Wundverbands, umfassend die Anwendung einer nach dem verfahren eines der vorhergehenden Ansprüche hergestellten Zusammellsetzung für die Wundheilung auf einem festen Träger.

**Revendications**
**Revendications pour les Etats contractants suivants: AT, BE, CH, DE, FR, GB, IT, LI, LU, NL, SE**

1. Composition cicatrisante des plaies de tissu mou, comprenant un mélange constitué :
   (a) de collagène fibrillaire
   (b) de 0,1 % à 10 % en poids, par rapport au collagène, d'héparine, d'un glycosaminoglycane du type héparine, ou d'un de leurs mélanges ; et
   (c) d'une quantité efficace d'au moins un facteur chimiotactique, de croissance ou de différenciation.

2. Composition selon la revendication 1, dans laquelle le facteur est le PDGF, le FGF ou un de leurs mélanges.

3. Composition selon la revendication 1 ou 2, dans laquelle le facteur est sous la forme de plaquettes non dégranulées ou d'une quantité équivalente de produit libéré des plaquettes.

4. Composition selon la revendication 3, dans laquelle le PDGF ou le FGF est du PDGF humain ou du FGF humain, et les plaquettes non dégranulées ou le produit libéré des plaquettes sont des plaquettes humaines ou du produit libéré de plaquettes humaines.

5. Composition selon l'une quelconque des revendications précédentes, dans laquelle le collagène fibrillaire est du collagène du Type 1, du collagène du Type III ou un de leurs mélanges.

6. Composition selon l'une quelconque des revendications précédentes, dans laquelle le collagène fibrillaire est du collagène fibrillaire reconstitué.

7. Pansement pour plaies, comprenant un support solide portant une composition selon l'une quelconque des revendications précédentes.

**Revendications pour les Etats contractants suivants : ES, GR**

1. Procédé pour la préparation d'une composition cicatrisante des plaies de tissu mou, consistant à combiner :
   (a) du collagène fibrillaire
   (b) de l'héparine, un glycosaminoglycane du type héparine, ou un de leurs mélanges, et
   (c) une quantité efficace d'au moins un facteur chimiotactique, de croissance ou de différenciation, telle que le poids de l'héparine, du glycosaminoglycane du type héparine, ou de leur mélange, soit de 0,1 % à 10 % en poids, par rapport au collagène.

2. Procédé selon la revendication 1, dans lequel le facteur est le PDGF, le FGF ou un de leurs mélanges.

3. Procédé selon la revendication 1 ou 2, dans lequel le facteur est sous la forme de plaquettes non dégranulées ou d'une quantité équivalente du produit libéré des plaquettes.

4. Procédé selon la revendication 3, dans lequel le PDGF ou le FGF est du PDGF humain ou du FGF humain, et les plaquettes non dégranulées ou le produit libéré des plaquettes sont des plaquettes humaines ou du produit libéré de plaquettes humaines.

5. Procédé selon l'une quelconque des revendications précédentes, dans lequel le collagène fibrillaire est du collagène du Type I, du collagène du Type III ou un de leurs mélanges.

6. Procédé selon l'une quelconque des revendications précédentes, dans lequel le collagène fibrillaire est du collagène fibrillaire reconstitué.

7. Procédé pour la préparation d'un pansement pour plaies, comprenant l'application sur un support solide d'une composition cicatrisante des plaies, préparée par le procédé selon l'une quelconque des revendications précédentes.

FIG. I

VASCULARIZATION— DAY 5

FIG. 2

RE-EPITHELIALIATION

FC+ PL

FC-H + PL

FIG. 3

FIG. 4